# EUROPEAN PATENT APPLICATION

(11) **EP 3 248 595 A1**
(43) Date of publication of application: **29.11.2017**
(21) Application number: 16740249.4
(22) Date of filing: 21.01.2016
(51) Int. Cl.: A61K 9/70, A61K 31/137, A61K 47/36, A61K 47/38

(54) **FILM PREPARATION**

(30) Priority: 22.01.2015 JP 2015010697
(71) Applicant: Nipro Corporation, Osaka-shi, Osaka 531-8510 (JP)
(72) Inventor: SHINKAI, Norihiro, Osaka-shi Osaka 531-8510 (JP); KAWAMURA, Naohisa, Osaka-shi Osaka 531-8510 (JP); SHIRAISHI, Hiroaki, Osaka-shi Osaka 531-8510 (JP); HAYASHIDA, Tomohiro, Osaka-shi Osaka 531-8510 (JP)
(74) Representative: Cabinet Plasseraud
(86) International application number: PCT/JP2016/051694
(87) International publication number: WO 2016/117642

(57) **Abstract**

The present invention addresses the problem of providing a film preparation that enables large quantities of medicinal products to be blended thereinto. The present invention provides a film preparation that contains a film base material, which contains a water-soluble polymer, and medicinal product-containing particles, which are supported on the film base material and which have a coating layer containing a hydrophobic coating agent on the outermost surface of the particles. The content of the medicinal product in the medicinal product-containing particles may be 0.1-95 mass% relative to the overall film preparation. The water-soluble polymer may include one or more types selected from the group consisting of hydroxypropyl cellulose, hydroxypropylmethyl cellulose and pullulan.

## Description

### TECHNICAL FIELD

The present invention relates to a film preparation.

### BACKGROUND ART

Conventionally, tablets, capsules, orally disintegrating films (also referred to as "ODF"), and the like have been marketed as oral pharmaceutical preparations. Of these, ODF have advantages such as easiness to take (see Patent Document 1, etc.).

Patent Document 1: Japanese Unexamined Patent Application, Publication No. 2011-207847

### DISCLOSURE OF THE INVENTION

### Problems to be Solved by the Invention

However, since ODF are usually prepared as a film preparation having a thin thickness, they had problems such as difficulty in blending large quantities of medicinal products. For example, when therapeutic agents for diseases that require administration of large quantities of medicinal products are prepared as an ODF, the necessary amount of the medicinal products cannot be supported on the ODF, and the medicinal products cannot be stably supported in the preparation due to crystallization, etc. of the medicinal products, and thus no preparation was obtained for practical use.

The present invention was made in view of these circumstances, and an object thereof is to provide a film preparation that enables large quantities of medicinal products to be blended thereinto.

### Means for Solving the Problems

The present inventors have found that the above problems can be solved by preparing a film preparation from medicinal product-containing particles, which have a coating layer containing a hydrophobic coating agent on the outermost surface of the particles, and a film base material, which contains a water-soluble polymer, thus completing the present invention. Specifically, the present invention provides the followings.
(1) A film preparation including a film base material containing a water-soluble polymer, and
   medicinal product-containing particles, which are supported on the film base material and which have a coating layer containing a hydrophobic coating agent on the outermost surface of the particles.
(2) The film preparation according to (1), wherein the content of the medicinal product in the medicinal product-containing particles is 0.1 to 95 mass% relative to the overall film preparation.
(3) The film preparation according to (1) or (2), wherein the water-soluble polymer includes one or more types selected from the group consisting of hydroxypropyl cellulose, hydroxypropylmethyl cellulose, and pullulan.
(4) The film preparation according to any one of (1) to (3), wherein the mean particle diameter of the medicinal product-containing particles is 1,000 µm or less.
(5) The film preparation according to any one of (1) to (4), wherein the medicinal product is water-insoluble.
(6) The film preparation according to any one of (1) to (5), wherein the ratio between the minimum thickness and the maximum thickness in the film preparation, minimum thickness:maximum thickness, is 1:1.5 to 1:5.
(7) A method for producing a film preparation, which includes a mixing step of mixing a film base material solution containing a water-soluble polymer and a solvent with medicinal product-containing particles having a coating layer containing a hydrophobic coating agent, and
   a film-preparing step of preparing a film from the composition obtained in the mixing step.
(8) The method according to (7), wherein the solvent is water or an aqueous ethanol solution.
(9) The film preparation according to (7) or (8), wherein the mean particle diameter of the medicinal product-containing particles is 1,000 µm or less.

### Effects of the Invention

According to the present invention, there is provided a film preparation that enables large quantities of medicinal products to be blended thereinto.

### BRIEF DESCRIPTION OF THE DRAWINGS

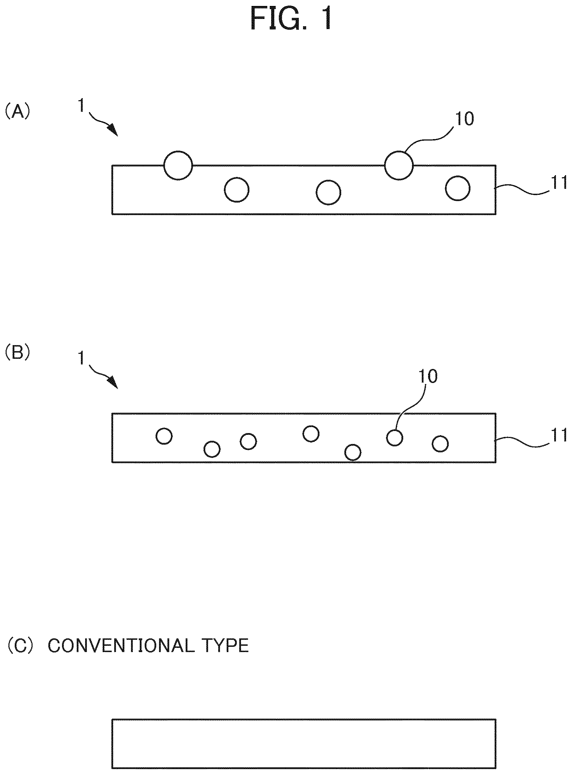

[Fig. 1] (A) A section view of a film preparation according to one embodiment of the present invention. (B) A section view of a film preparation according to another embodiment of the present invention. (C) A section view of a conventional film preparation.

### PREFERRED MODE FOR CARRYING OUT THE INVENTION

Hereinafter, embodiments of the present invention will be described. The present invention is not limited to the following embodiments.

### <Film Preparation>

A film preparation of the present invention is prepared from a film base material containing a water-soluble polymer, and medicinal product-containing particles having a coating layer containing a hydrophobic coating agent on the outermost surface of the particles. In the film preparation of the present invention, the medicinal product-containing particles are supported on the film base material. As used herein, "medicinal product-containing particles are supported on the film base material" means that at least part of the surface of the medicinal product-containing particles is in contact with the film base material. As used herein, "hydrophobic" and "insoluble" mean that, regarding the solubility in water, the amount of water necessary for dissolving 1 g of a solute at 20°C is 100 mL or more. "Water-soluble" means that, regarding the solubility in water, the amount of water necessary for dissolving 1 g of a solute at 20°C is less than 5 mL.

A conventional film preparation is obtained by dispersing medicinal products together with a film base material in a solvent, followed by spreading and drying of the obtained dispersion. However, in such film preparation, the amount of medicinal products that can be blended depends on the solubility of the medicinal products in a solvent or a dispersion. For example, in a film preparation containing a water-soluble polymer in a film base material, it is difficult to blend water-insoluble medicinal products since an aqueous solvent is used for the preparation. In this case, it is difficult to prepare a film preparation, and even if a film preparation can be prepared, crystallization or precipitation, etc. of the medicinal products occurs, and thus no film preparation can be obtained for practical use.

On the other hand, in the present invention, medicinal products are prepared as medicinal product-containing particles having a coating layer containing a hydrophobic coating agent on the outermost surface of the particles, and the medicinal product-containing particles are dispersed in a solvent together with a film base material containing a water-soluble polymer, and a film preparation is prepared from the obtained dispersion. Since the medicinal product-containing particles and the film base material have different affinity for a solvent, the medicinal product-containing particles are dispersed in the film base material in the film preparation of the present invention. Since the medicinal products are encapsulated in the medicinal product-containing particles, crystallization or precipitation, etc. is extremely less likely to occur. Furthermore, in the film preparation of the present invention, since the amount of medicinal products that can be blended does not depend on the solubility of the medicinal products in a solvent, etc., crystallization or precipitation, etc. of the medicinal products is extremely less likely to occur even when larger quantities of the medicinal products than conventional quantities are blended, and thus the medicinal products can be stably supported in the film preparation.

### (Medicinal product-Containing Particles)

There is no particular limitation on the structure of the medicinal product-containing particles as long as the outermost surface is covered by a coating layer containing a hydrophobic coating agent. Examples of the structure include (1) a structure including medicinal product-containing particles having no core and a coating layer containing a hydrophobic coating agent, and (2) a structure including core microparticles (carriers such as crystalline cellulose, D-mannitol, lactose, and purified sucrose), a medicinal product layer, and a coating layer containing a hydrophobic coating agent. The medicinal product-containing particles may or may not appropriately contain a publicly known bitterness-masking layer, controlled-release layer, enteric-coated layer, gastric-coated layer, or the like, according to the objective of obtaining the particles.

There is no particular limitation on the medicinal product contained in the medicinal product-containing particles. In the film preparation of the present invention, large quantities of medicinal products can be supported in the film preparation regardless of the properties of the medicinal products. For example, in a conventional film preparation, if the solubility of medicinal products in a solvent, etc. is low, the medicinal products could not be prepared as a film preparation. Particularly, medicinal products that are insoluble in a water-containing solvent (e.g., water, an aqueous ethanol solution) was difficult to be prepared as a film preparation. However, in the film preparation of the present invention, even medicinal products having low solubility in a solvent, etc. can also be easily prepared as a film preparation.

There is no particular limitation on the medicinal product contained in the medicinal product-containing particles as long as it is a medicinal product that can be orally administered. Specific examples thereof include sedatives, expectorants, cathartics, anticancer agents, antidiabetic agents, antiparkinsonian agents, antidepressants, tranquilizers, antidementia agents, antihypertensives, lipid-lowering agents, antimigraine agents, antiosteoporosis agents, antihypotensives, antitussives, therapeutic agents for peptic ulcer, therapeutic agents for pollakisuria/dysuria, therapeutic agents for urinary incontinence, antiulcer agents, antiallergic agents, 5-HT3 receptor antagonists (antiemetics), and the like.

There is no particular limitation on the coating layer that covers the outermost surface of the medicinal product-containing particles as long as it is a coating layer prepared from a hydrophobic coating agent, etc. As the hydrophobic coating agent, for example, usually water-insoluble components, etc., which are used in preparation of medicinal products, etc. can be used. Examples of such components are as follows.
(1) Controlled-release coating agents (specifically, ethyl cellulose and an ethyl acrylate-methyl methacrylate-trimethylammonium ethyl methacrylate chloride copolymer, and a dispersion including one or more selected from the group consisting of ethyl cellulose, an ethyl acrylate-methyl methacrylate-trimethylammonium ethyl methacrylate chloride copolymer, and an ethyl acrylate-methyl methacrylate copolymer in the form of latex.)
(2) Enteric coating agents (specifically, a solution in which one or more selected from the group consisting of hydroxypropylmethyl cellulose phthalate, hydroxypropylmethyl cellulose acetate succinate, a methacrylate-methyl methacrylate copolymer, a methacrylate-ethyl methacrylate copolymer, and a methacrylate-ethyl acrylate copolymer are dissolved using an organic solvent (e.g., methanol, ethanol, isopropanol, dichloromethane), or an aqueous dispersion including them in the form of latex.)
(3) Gastric coating agents (specifically, polyvinyl acetal diethylamino acetate, a methyl methacrylate-butyl methacrylate-dimethylaminoethyl methacrylate copolymer, and the like.)

Use of controlled-release coating agents can impart sustained-release properties to a film preparation. Use of enteric coating agents can impart enteric properties to a film preparation. Use of gastric coating agents can impart bitterness-masking effects to a film preparation. A film preparation to which the above effects are imparted can also be referred to as a film preparation that contains medicinal product-containing microparticles having functionalities such as enteric properties, sustained-release properties, or masking.

The coating layer that covers the outermost surface of the medicinal product-containing particles may contain components that are usually blended into the coating layer of the medicinal products, other than the above hydrophobic coating agents. Examples of such components include water-soluble components (e.g., macrogol, hypromellose (also called hydroxypropylmethyl cellulose), hydroxypropyl cellulose). Regarding the proportion of these components in the coating layer, the total amount of these components is preferably lower than the blending amount of the hydrophobic coating agent, in terms of the fact that a film preparation that enables large quantities of medicinal products to be blended thereinto is easily obtained.

Binders, saccharides, sugar alcohols, disintegrators, lubricants, or the like may or may not be appropriately blended into the medicinal product-containing particles, according to the objective of obtaining the particles.

There is no particular limitation on the amount of medicinal products to be blended into the medicinal product-containing particles, but, as mentioned above, since in the film preparation of the present invention, even large amounts of medicinal products that constitute the medicinal product-containing particles are less likely to cause crystallization or precipitation, etc. and large quantities of medicinal products can be blended, the amount may be 0.1 mass% or more, preferably 25 mass% or more, and more preferably 40 mass% or more relative to the overall film preparation. The amount of medicinal products may be 0.1 mass% or more, preferably 10 mass% or more, and more preferably 35 mass% or more relative to the overall medicinal product-containing particles.

On the other hand, the film preparation of the present invention can be satisfactorily prepared even when a similar amount of medicinal products to the amount blended into a conventional film preparation is blended, and thus the amount of medicinal products to be blended into the medicinal product-containing particles may be 95 mass% or less, preferably 85 mass% or less, and more preferably 75 mass% or less relative to the overall film preparation. The amount of medicinal products may be 95 mass% or less, preferably 85 mass% or less, and more preferably 60 mass% or less relative to the overall medicinal product-containing particles.

Into the film preparation of the present invention, 0.01 to 50.0 mg/cm² per area of the film preparation of medicinal products may be blended, and preferably 0.1 to 20 mg/cm² of medicinal products may be blended.

In a conventional film preparation, for example, only about 20 mass% of medicinal products could be blended relative to the overall film preparation, while in the film preparation of the present invention, the amount same as to 10-fold higher than that amount can be stably blended.

There is no particular limitation on the method for producing the medicinal product-containing particles, and publicly known granulation methods or coating methods can be used. Examples thereof include a method in which a solution in which a hydrophobic coating agent is dissolved is sprayed for coverage over medicinal product-containing particles obtained by a method in which medicinal products and microcrystalline cellulose are mixed and water is added, followed by mixing and stirring (see Japanese Patent No. 5511663, etc.), a method in which a solution in which a hydrophobic coating agent is dissolved is sprayed for coverage over medicinal product-containing microparticles obtained by layering medicinal products on core microparticles (see Japanese Unexamined Patent Application, Publication No. 2014-101332, etc.), and the like.

The mean particle diameter of the medicinal product-containing particles can be appropriately adjusted according to a formulation technique to be used, and is preferably 1,000 µm or less, more preferably 400 µm or less, and most preferably 300 µm or less, in terms of the fact that graininess in the oral cavity can be reduced.

The film preparation of the present invention can be prepared in a state in which the medicinal product-containing particles are almost uniformly dispersed in the film base material regardless of the particle diameter of the medicinal product-containing particles since the affinity of the medicinal product-containing particles for a solvent differs from the affinity of the film base material for a solvent, and thus, as shown in Fig. 1(A), the film preparation can be prepared so that part of the surface of the medicinal product-containing particles is exposed from the surface of the film base material. Therefore, the particle diameter of the medicinal product-containing particles can be set at a larger value relative to the film thickness.

### (Film Base Material)

There is no particular limitation on the film base material as long as it includes a water-soluble polymer. As the water-soluble polymer, usually water-soluble components, etc., which are used in pharmaceuticals, etc., can be used. Examples of the water-soluble polymer include starches, modified starches, polysaccharides, proteins, peptides, cellulose derivatives, synthetic polymers, and the like.

Examples of the water-soluble polymer include the following compounds: synthetic polymer compounds such as hydroxypropyl cellulose (HPC), hydroxypropylmethyl cellulose (HPMC), pullulan, polyvinyl alcohol, polyvinylpyrrolidone, carboxyvinyl polymer, hydroxyethyl cellulose, methylcellulose, ethyl cellulose, low substituted hydroxypropyl cellulose, crystalline cellulose, sodium carboxymethyl cellulose, calcium carboxymethyl cellulose, carboxymethyl cellulose, sodium carboxymethyl starch, and crystalline cellulose, and sodium alginate, dextran, casein, gelatin, pectin, guar gum, xanthan gum, tragacanth gum, Acacia gum, gum arabic, corn starch, pregelatinized starch, and the like.

Of the above water-soluble polymers, a water-soluble polymer that includes one or more types selected from the group consisting of hydroxypropyl cellulose, hydroxypropylmethyl cellulose, and pullulan is preferable, in terms of the fact that the medicinal product-containing particles can be particularly stably supported in the film preparation.

When two or more types of water-soluble polymers are included in the film base material, the blending ratio can be appropriately adjusted according to effects to be obtained.

As the water-soluble polymer, a combination of hydroxypropyl cellulose and pullulan or a combination of hydroxypropyl cellulose and hydroxypropylmethyl cellulose is preferable, in terms of the fact that the strength and disintegration of a film preparation obtained are satisfactory.

When the water-soluble polymer is a combination of hydroxypropyl cellulose (HPC) and pullulan, the blending ratio (mass ratio), HPC:pullulan, is preferably 2:6 to 6:2, in terms of the fact that the strength and disintegration are particularly satisfactory.

When the water-soluble polymer is a combination of hydroxypropyl cellulose (HPC) and hydroxypropylmethyl cellulose (HPMC), the blending ratio (mass ratio), HPC:HPMC, is preferably 1:3 to 1:1, in terms of the fact that the strength and disintegration are particularly satisfactory.

Binders, corrigents (e.g., sugars such as saccharin sodium hydrate, flavors), plasticizers (e.g., polyethylene glycol), perfumes, colorants, preservatives, antioxidants, stabilizers, surfactants, or the like may or may not be appropriately blended into the film base material, according to the objective of obtaining the film base material.

There is no particular limitation on the blending amount of the water-soluble polymer in the film base material as long as the medicinal product-containing particles are supported and a film preparation can be prepared, and the blending amount may be 50 to 100 mass% relative to the overall film base material. The blending amount may be 10 to 95 mass% relative to the overall film preparation. The film base material may be composed of a water-soluble polymer.

In the film preparation of the present invention, a publicly known layer, etc. may be further appropriately included.

### <Method for Producing Film Preparation of Present Invention>

The film preparation of the present invention can be obtained by using the above medicinal product-containing particles and the above film base material to perform preparation by a publicly known film production method, etc. Examples of preferable production methods include a method in which a film base material solution is obtained by mixing a water-soluble polymer with a solvent, and the film base material solution is mixed with the above medicinal product-containing particles, and then a film is prepared from the preparation bulk thus obtained (a composition before preparation of a film preparation by drying). Examples of the method for preparing a film from a preparation bulk include a method in which a preparation bulk is spread into a thin film form to dry a solvent.

As a solvent used for preparation of a film preparation, a solvent which has a less content of plasticizers (e.g., polyethylene glycol), etc. and which contains much water is preferable so as not to dissolve a coating layer covering the medicinal product-containing particles. Specifically, a solvent made of water and an aqueous ethanol solution are preferable. The aqueous ethanol solution preferably contains 60% or less of ethanol so as not to damage the coating layer.

The amount of a solvent used is not particularly limited, and may be 50 to 90 mass% relative to the film base material solution. Although a solvent is used for preparation of a film preparation and is not contained in the obtained film preparation in principle, the present invention does not exclude that a solvent is contained in the film preparation insofar as the function as a film preparation is not impaired.

The preparation bulk in the present invention is preferably composed of the medicinal product-containing particles, the film base material, and water, in terms of the fact that the film preparation of the present invention is easily prepared.

In the film preparation of the present invention, the medicinal product-containing particles are supported in a state in which they are dispersed in the film base material. Examples of a section view of the film preparation of the present invention are shown in Figs. 1(A) and 1(B). As shown in Fig. 1(A), part of the surface of the medicinal product-containing particles may be exposed from the surface of the film base material. Since the film preparation of the present invention can be in the form as shown in Fig. 1(A), the particle diameter of the medicinal product-containing particles can be set at a large value and large quantities of medicinal products can be blended. On the other hand, the film preparation of the present invention can also be in the form as shown in Fig. 1(B) and prepared without exposing the surface of the medicinal product-containing particles from the surface of the film base material.

When the film preparation of the present invention is in the form in which part of the surface of the medicinal product-containing particles is exposed from the surface of the film base material as shown in Fig. 1(A), the ratio between the minimum thickness and the maximum thickness of the film preparation, minimum thickness:maximum thickness, may be 1:1.5 to 1:5.

The shape, etc. of the film preparation of the present invention is not particularly limited, and can be appropriately adjusted according the use, etc. For example, the thickness of the film may be 100 to 300 µm. The size of the film preparation may be 0.09 to 16.0 cm². The shape of the film preparation may be quadrilateral, round, or the like.

### EXAMPLES

The present invention will be described in more details by way of Examples, but the present invention is not limited to these Examples.

### <Example 1: Preparation of Film Preparation>

Using ambroxol hydrochloride as a medicinal product, a film preparation was prepared in accordance with the following methods.

### (Preparation of Medicinal product-Containing Particles)

Based on the following methods, medicinal product-containing particles were prepared.

### (1) Preparation of Medicinal product-Layering Particles

After addition of 232 g of ambroxol hydrochloride, 56.8 g of povidone (trade name: Kollidon 30, manufactured by BASF Japan Ltd.), and 56.8 g of crospovidone (trade name: Kollidon CL-M, manufactured by BASF Japan Ltd.) to 1,963 g of purified water, the substances were dispersed by stirring to prepare Layering Solution A. After addition of 232 g of ambroxol hydrochloride and 56.8 g of povidone (trade name: Kollidon, manufactured by BASF Japan Ltd.) to 1,963 g of purified water, the substances were dispersed by stirring to prepare Layering Solution B. After Layering Solution A was sprayed over 500 g of powder crystalline cellulose (trade name: Celphere CP102, manufactured by Asahi Kasei Chemicals Corporation) using a tumbling fluidized bed coating granulator (manufactured by Powrex Corp.: MP-01), Layering Solution B was sprayed to perform coating of the surface. Next, the particles obtained by drying were sieved through a 42-mesh sieve and a 150-mesh sieve to obtain medicinal product-layering microparticles.

### (2) Preparation of Seal-Coated Microparticles

After addition of 23.8 g of hypromellose (trade name: TC-5E, manufactured by Shin-Etsu Chemical Co., Ltd.) and 10.2 g of sucralose (trade name: Sucralose P, manufactured by San-Ei Gen F.F.I., Inc.) to 647 g of purified water, the substances were dissolved by stirring to prepare a seal-coating solution. The seal-coating solution was sprayed over 1,135 g of the medicinal product-layering particles prepared above using a tumbling fluidized bed coating granulator (manufactured by Powrex Corp.: MP-01) to perform coating of the surface, and the particles thus obtained were sieved through a 42-mesh sieve and a 150-mesh sieve to obtain seal-coated microparticles.

### (3) Preparation of Sustained-Release Microparticles

After addition of 2,760 g of an 80% ethanol solution to 183 g of ethyl cellulose (trade name: ETHOCEL 10, manufactured by The Dow Chemical Company) and 57.1 g of hypromellose (trade name: TC-5R, manufactured by Shin-Etsu Chemical Co., Ltd.), the substances were dissolved by stirring to prepare a controlled-release coating solution. The controlled-release coating solution was sprayed over 500 g of the seal-coated microparticles prepared above using a tumbling fluidized bed coating granulator (manufactured by Powrex Corp.: MP-01) to perform coating of the surface, and the particles thus obtained were dried and then sieved through a 42-mesh sieve and a 150-mesh sieve to obtain sustained-release microparticles.

### (4) Preparation of Overcoating Microparticles

After addition of 15.2 g of hypromellose (trade name: TC-5R, manufactured by Shin-Etsu Chemical Co., Ltd.) and 60.8 g of macrogol (trade name: Macrogol 6000, manufactured by NOF CORPORATION) to 600.4 g of purified water, the substances were dissolved to prepare Overcoating Solution A. After addition of 40.8 g of ethyl cellulose (trade name: ETHOCEL 7, manufactured by The Dow Chemical Company) and 17.5 g of hypromellose (trade name: TC-5R, manufactured by Shin-Etsu Chemical Co., Ltd.) to an 80% ethanol solution, the substances were dissolved by stirring to prepare Overcoating Solution B. Overcoating Solution A was sprayed over 500 g of the sustained-release microparticles prepared above using a tumbling fluidized bed coating granulator (manufactured by Powrex Corp.: MP-01), and then Overcoating Solution B was sprayed to perform coating of the surface. Next, the particles obtained by drying were sieved through a 42-mesh sieve and a 150-mesh sieve to obtain overcoating microparticles. The overcoating microparticles correspond to "medicinal product-containing particles, which have a coating layer containing a hydrophobic coating agent on the outermost surface of the particles". Ethyl cellulose corresponds to the "hydrophobic coating agent".

### (Preparation of Film Preparation)

Water and a water-soluble polymer were added to a container, followed by stirring, and then the medicinal product-containing microparticles prepared above were added and dispersed by stirring to obtain a drug solution. The drug solution thus obtained was spread into a thin film form in the frame prepared with a PET film using a coating test machine, and moisture in the drug solution was dried to obtain a film preparation. In this Example, as the water-soluble polymer, pullulan and hydroxypropyl cellulose were used in the proportion (mass ratio), pullulan:hydroxypropyl cellulose, of 2:6.

The mass of the medicinal product-containing particles obtained was 135 mg per particle, and the mass ratio between the hydrophobic coating agent and the medicinal product was about 1:1. The film preparation of Example 1 was in the form in which part of the surface of the medicinal product-containing particles was exposed from the surface of the film base material (see Fig. 1(A)).

### <Comparative Example 1: Preparation of Conventional Film Preparation>

Using ambroxol hydrochloride as a medicinal product, a film preparation was prepared in accordance with the following methods.

### (Preparation of Film Preparation)

Water and a water-soluble polymer were added to a container, followed by stirring, and then ambroxol hydrochloride was dissolved in the solution obtained. The solution thus obtained was applied in a uniform thickness, and then dried to obtain a film preparation. In this Example, as the water-soluble polymer, pullulan and hydroxypropyl cellulose were used in the proportion (mass ratio), pullulan:hydroxypropyl cellulose, of 2:6.

In the film preparation of Comparative Example 1, the medicinal product was dissolved in the film base material, and no unevenness, etc. was observed on the surface (see Fig. 1(C)).

Data on the area, etc. of each film preparation obtained are shown in Table 1. The thickness of the film preparation of Example 1 was measured at two points including a part in which the surface of the medicinal product-containing particles is not exposed from the surface of the film base material (120 µm) and a part having the maximum thickness in which the surface of the medicinal product-containing particles is exposed from the surface of the film base material (300 µm).

**[Table 1]**

| | Example 1 | Comparative Example 1 |
|---|---|---|
| Area per film preparation | 6cm² | 6cm² |
| Thickness of film preparation | 120µm to 300µm | 120µm |
| Mass of film preparation | 180mg | 72mg |
| Proportion of medicinal product in film preparation | 75 mass% | 20.8 mass% |
| Content of medicinal product in film preparation | 67.5mg | 15.0mg |
| Medicinal -product-masking effects | Satisfactorily maintained | None |
| Symptoms after oral administration | No symptoms | Numbness |

As shown in Table 1, in the film preparation of the present invention of Example 1 (content of the medicinal product: 67.5 mg), a 4.5-fold higher amount of the medicinal product could be blended, compared with that of the conventional film preparation shown in Comparative Example 1 (content of the medicinal product: 15.0 mg). In the film preparation of the present invention, no crystallization, etc. of the medicinal product was observed, and the medicinal product could be uniformly supported in the film preparation. When the same amount of the medicinal product as in Example 1 was blended in the conventional film preparation, the medicinal product could not be supported in the preparation, and formulation was impossible. Therefore, according to the present invention, it is possible to stably support a significantly higher amount of the medicinal product than that of the conventional film preparation in the film preparation.

Furthermore, the film preparation of the present invention had satisfactory masking effects on bitterness, etc. of the medicinal product. This tendency was observed regardless of the blending amount of the medicinal product in the film preparation. On the other hand, the conventional film preparation showed no masking effects on bitterness, etc. of the medicinal product, and suffering or numbness occurred after intake.

Although data are not shown, the film preparation of Example 1 had superior strength, while the film preparation of Comparative Example 1 was brittle and had insufficient strength.

### <Examples 2 to 6: Investigation of Types of Water-Soluble Polymers contained in Film Base Material>

A film preparation was prepared in the same manner as in Example 1, except that the following water-soluble polymers were used and the proportion of the medicinal product in the film preparation was 50 mass%. Hereinafter, "HPC" means hydroxypropyl cellulose, and "HPMC" means hydroxypropylmethyl cellulose.
(Water-Soluble Polymer used and its Blending Ratio (Mass Ratio))
Example 2 pullulan:HPC = 1:3
Example 3 pullulan:HPC = 3:1
Example 4 HPC:HPMC = 1:3
Example 5 HPC:HPMC = 3:5
Example 6 HPC:HPMC = 1:1

Each film preparation obtained was bended by hand, and the strength was evaluated. Each film preparation obtained was put into the oral cavity, and the disintegration was evaluated. The results are shown in Table 2.

**[Table 2]**

| | Sensory evaluation | |
|---|---|---|
| | Strength | Disintegration |
| Example 2 | Satisfactory | Satisfactory |
| Example 3 | Satisfactory | Satisfactory |
| Example 4 | Satisfactory | Satisfactory |
| Example 5 | Satisfactory | Satisfactory |
| Example 6 | Satisfactory | Satisfactory |

As shown in Table 1, both strength and disintegration of each film preparation were satisfactory. Satisfactory strength of the film preparation means that even when the film preparation was bended, it was flexible and did not crumble or break. Satisfactory disintegration of the film preparation means that when the film preparation was put into the oral cavity, it was dissolved or collapsed by saliva, etc. in about 10 seconds, and could be rapidly orally taken.

### EXPLANATION OF REFERENCE NUMERALS

1 Film preparation
10 Medicinal product-containing particle
11 Film base material

## Claims

1. A film preparation comprising a film base material containing a water-soluble polymer, and
medicinal product-containing particles, which are supported on the film base material and which have a coating layer containing a hydrophobic coating agent on the outermost surface of the particles.

2. The film preparation according to claim 1, wherein the content of a medicinal product in the medicinal product-containing particles is 0.1 to 95 mass% relative to the overall film preparation.

3. The film preparation according to claim 1 or 2, wherein the water-soluble polymer includes one or more types selected from the group consisting of hydroxypropyl cellulose, hydroxypropylmethyl cellulose, and pullulan.

4. The film preparation according to any one of claims 1 to 3, wherein the mean particle diameter of the medicinal product-containing particles is 1,000 µm or less.

5. The film preparation according to any one of claims 1 to 4, wherein the medicinal product is water-insoluble.

6. The film preparation according to any one of claims 1 to 5, wherein the ratio between the minimum thickness and the maximum thickness in the film preparation, minimum thickness:maximum thickness, is 1:1.5 to 1:5.

7. A method for producing a film preparation, which comprises a mixing step of mixing a film base material solution containing a water-soluble polymer and a solvent with medicinal product-containing particles having a coating layer containing a hydrophobic coating agent, and
a film-preparing step of preparing a film from a composition obtained in the mixing step.

8. The production method according to claim 7, wherein the solvent is water or an aqueous ethanol solution.

9. The film preparation according to claim 7 or 8, wherein the mean particle diameter of the medicinal product-containing particles is 1,000 µm or less.
